(19) Europäisches Patentamt
European Patent Office
Office européen des brevets

(11) Veröffentlichungsnummer: **0 339 502 B1**

(12) **EUROPÄISCHE PATENTSCHRIFT**

(45) Veröffentlichungstag der Patentschrift: **08.12.93**

(51) Int. Cl.⁵: **A61M 1/16**, A61L 33/00, B01D 61/24

(21) Anmeldenummer: **89107190.4**

(22) Anmeldetag: **21.04.89**

# (54) Verfahren zur Modifizierung von cellulosischen Dialysemembranen zur Verbesserung der Biocompatibilität und Vorrichtung zur Durchführung des Verfahrens.

(30) Priorität: **28.04.88 DE 3814326**

(43) Veröffentlichungstag der Anmeldung:
**02.11.89 Patentblatt 89/44**

(45) Bekanntmachung des Hinweises auf die Patenterteilung:
**08.12.93 Patentblatt 93/49**

(84) Benannte Vertragsstaaten:
**DE FR IT**

(56) Entgegenhaltungen:
**EP-A- 0 201 378**
**EP-A- 0 266 795**
**US-A- 4 000 126**
**US-A- 4 145 295**

(73) Patentinhaber: **Akzo N.V.**
**Postbus 9300**
**Velperweg 76**
**NL-6800 SB Arnhem(NL)**

(72) Erfinder: **Diamantoglou, Michael, Dr.**
**Kolpingstrasse 4**
**D-8765 Erlenbach/Main(DE)**

(74) Vertreter: **Fett, Günter**
**Akzo Patente GmbH,**
**Postfach 10 01 49**
**D-42097 Wuppertal (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft ein Verfahren zur chemischen Modifizierung von cellulosischen Dialysemembranen sowie solchen auf Chitinbasis in Form von Flachfolien, Schlauchfolien oder Hohlfäden zur Verbesserung der Biokompatibilität, ausgedrückt durch eine Reduzierung der C5a-Komplementaktivierung und einer erhöhten β2-Microglobulin-Adsorption.

In der US-A-3 745 202 und der DE-A-2 300 496 werden Verfahren zur Herstellung asymmetrischer Membranen von Cellulosederivaten mit Ester- und/oder Ethergruppen beschrieben.

Aus der DE-B-27 05 735 ist eine Dialysemembran für die Hämodialyse mit daran chemisch gebundenen antithrombogenen Verbindungen bekannt, wobei die Dialysemembran aus zwei oder mehreren Schichten einer aus Cuoxamcelluloselösungen regenerierten Cellulose besteht, die jeweils aus getrennt gespeisten Schlitzen einer Spinndüse erhalten worden ist, die antithrombogene Wirkstoffe chemisch gebunden enthält.

Die DE-A-27 48 858 vom 31.10.77 beschreibt die Herstellung von antithrombogenen Polymermaterialien, die nach folgender Verfahrensweise hergestellt werden:

- Umsetzung von reaktiven Polymeren mit synthetischen fibrinolytischen Verbindungen (kovalente Bindung)
- Behandlung von Anionenaustauschgruppen enthaltenden Polymeren mit einer synthetischen fibrinolytischen Verbindung (ionische Bindung)
- Behandlung von Polymerstoffen mit Lösungen von synthetischen fibrinolytischen Verbindungen (Adsorption).

Eine solche Membranmodifizierung scheidet aus, weil biokompatibilitätsverbessernde Verbindungen, die nur adsorptiv an das Polymer gebunden sind, während der Dialyse in die Blutbahn gelangen können.

Das US-Patent US-A-3 475 410 vom 28.10.69 und die Veröffentlichung in Vol. XII Trans. Amer. Soc. Artif. Int. Organs, 1966, S. 139 - 150 beschreiben antithrombogene Cellulosemembranen, die durch Behandlung der Cellulose zunächst mit Ethylenimin und anschließend mit Heparin erhalten werden. Nach unseren Untersuchungen weisen aber mit Ethylaminogruppen modifizierte Membranen eine schlechtere Biokompatibilität als unmodifizierte auf.

In der EP-A-0 232 442 werden Cellulose-Membranen beschrieben, bei denen die Seite der Membran, die bei der Dialyse Kontakt zum Blut hat, mit einer polymeren Substanz bedeckt ist. Es handelt sich dabei um Verbindungen auf der Basis von Vinylmonomeren mit einer Aminogruppe in der Seitenkette. Die Menge an Beschichtungs-(Bedeckungs-)-material liegt im ppm-Bereich, so daß eine durchgehende Modifizierung der Membran ausgeschlossen ist. Die Wirksamkeit der nach der EP-A-0 232 442 hergestellten Membranen liegt hauptsächlich in einer Reduzierung der Leukopenie, während die Effektivität der Membran bei der Reduzierung der Komplementaktivierung gering bleibt.

Die japanischen Anmeldungen JP-A-57 162 701 und JP-A-57 162 702 beanspruchen ebenfalls antithrombogene Cellulosemembranen. Diese werden durch Aufpfropfen von Vinylmonomeren an Cellulose oder Cellulosederivate und nachfolgende Heparinisierung hergestellt. Neben der Aufpfropfreaktion findet aber bekanntlich auch eine Homopolymerisation statt. Obwohl zwischen Homopolymerisat und Cellulose keine feste Bindung besteht, kann dieses trotz intensiven Waschens nicht vollständig aus der Membran entfernt werden. Deshalb können während der Blutdialyse stets kleine Mengen des Homopolymerisats in die Blutbahn gelangen.

Die japanische Patentanmeldung JP-A-60-203 265 beschreibt hochmolekulare Celluloseprodukte zur Herstellung von medizinischen Instrumenten mit Anticoagulanteigenschaften. Es handelt sich dabei um Mischungen polykationischer und polyanionischer Cellulosederivate, die üblicherweise durch Vermischen entsprechender Polymerlösungen erhalten werden. Derartige wasserunlösliche Salze sind als Membranmaterialien ungeeignet, da stets die Gefahr besteht, daß sie durch Umsalzeffekte in eine wasserlösliche oder in Wasser stark quellbare Verbindung umgewandelt werden.

Es ist aber auch bereits in der DE-A-17 20 087 vorgeschlagen worden, dadurch daß das Polymermaterial der Membran mit einem Alkylhalogenid umgesetzt und danach das erhaltene Material mit einem Alkalisalz einer antithrombogenen Verbindung mit kationischem Rest (z.B. Heparin oder eine Heparinoidverbindung) umgesetzt wird, die Gefahr der Gerinnung des Blutes zu verringern. Zu den möglichen Alkylhalogeniden werden dabei auch Halogenalkyldialkylamine gerechnet. Cellulose und Celluloseacetat zählen zu den möglichen Polymeren.

Eine antithrombogene Wirkung dieser bekannten Dialysemembranen wird nur beobachtet, wenn der Substitutionsgrad der modifizierten Cellulose hoch ist, d.h. größer als mindestens 0,1, und in einem gesonderten Schritt eine Vorheparinisierung mit relativ hoher Heparinkonzentration (Lösungen mit 0,1 bis 1 Gew.%) durchgeführt wird.

Aus der DE-A-33 41 113 ist eine Dialysemembran in Form von Flachfolien, Schlauchfolien oder Hohlfäden aus regenerierter Cellulose bekannt, bei der mindestens an einer Membran-Oberfläche über an die Cellulose chemisch gebundene Brückenbildner polymere Säuren chemisch gebunden sind. Abgesehen davon, daß die Herstellung, obwohl sie in einer Nachbehandlung erfolgt, relativ aufwendig ist, ist die Wirksamkeit im wesentlichen auf eine Reduzierung der Leucopenie beschränkt. Wegen der großen Moleküle der polymeren Säuren erfolgt die Anbindung über die Brückenbildner lediglich an der Oberfläche der Membran.

Weiterhin ist auch aus der DE-A-34 38 531 eine Dialysemembran bekannt, bei der Isocyanatpräpolymere an die Cellulose gebunden werden. Die Wirksamkeit ist in ähnlicher Weise beschränkt, wie bei der vorstehend aufgeführten, mit polymeren Säuren modifizierten Cellulosemembran.

Aus der DE-A-35 24 596 ist bereits eine Dialysemembran mit verbesserter Biocompatibilität bekannt, die sich dadurch auszeichnet, daß der mittlere Substitutionsgrad einer modifizierten Cellulose 0,02 bis 0,07 beträgt. Vorzugsweise enthält die bekannte Dialysemembran aus modifizierter Cellulose solche modifizierte Cellulose, die eine durch die Formel

Cellulose-R′-X-Y

wiedergegebene Struktur aufweist, wobei

X für -NR″- und/oder $-\overset{\oplus}{N}R''_2$ - und/oder -S- und/oder -SO- und/oder $-SO_2$- und/oder

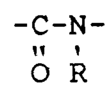

und/oder -CO-O- und/oder -O-,

Y für -R und/oder $-NR_2$ und/oder $-Si(OR'')_3$ und/oder $-SO_3H$ und/oder -COOH und/oder $-PO_3H_2$ und/oder $-\overset{\oplus}{N}HR_2$ bzw. deren Salze,

R′ für eine Alkylengruppe und/oder Cycloalkylengruppe und/oder Arylengruppe mit insgesamt 1 bis 25 C-Atomen,

R″ für ein Wasserstoffatom oder R und

R für eine Alkylgruppe mit 1 bis 5 C-Atomen und/oder eine Cycloalkylgruppe und/oder Arylgruppe steht.

Diese bekannte Dialysemembran war bereits in der Lage, Blutgerinnung, Leucopenie und Komplementaktivierung in erheblichem Umfange zu reduzieren. Eine Adsorption von Beta-2-Mikroglobulin konnte jedoch in nennenswertem Umfange nicht erreicht werden.

In der DE-A-37 23 897.3 werden Cellulosederivate mit der allgemeinen Formel

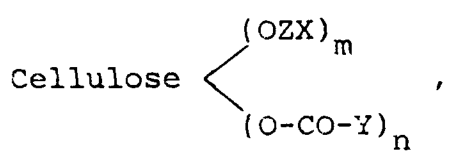

worin

-Z- einen gegebenenfalls substituierten Alkylen-, Alkenylen-, Alkinylen-, Cycloalkylen- oder Benzylen- oder Xylylenrest,

X -H, $-NR_2$, $-\overset{\oplus}{N}R_3$, -CN, -COOH, $-SO_3H$, $-PO(OR)_2$, $-CONR_2$ oder $-Si(OR)_3$ bedeuten,

wobei

R ein Wasserstoffatom oder eine Alkyl- oder Alkenylgruppe mit 1 bis 25 C-Atomen, Cycloalkyl-, Tolyl- oder Phenylgruppe bedeutet

und

Y eine gegebenenfalls substituierte Alkyl-, Alkenyl-, Alkinylgruppe mit 1 bis 36 C-Atomen, Cycloalkylgruppe oder eine Phenyl-, Tolyl- oder Benzylgruppe oder ein

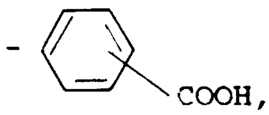

$-(CH_2)_r-COOH$,

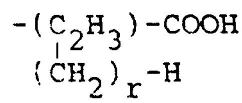

oder (-CH = CH-COOH)Rest oder NH-R-Rest ist und R die gleiche Bedeutung wie oben hat und

r = 1 - 20
m = 0 - 2,5
n = 0,2 bis 2,95

mit der Maßgabe, daß bei m = o n ≧ 1,55 ist, wenn Y ein Alkyl-Rest mit 1 - 5 C-Atomen, ein $-(CH_2)_r$-COOH-Rest mit r = 0, 1 oder 2 oder ein Rest der Phthalsäure ist, sowie der Polymerisationsgrad mehr als 400 beträgt, und die herstellbar sind durch homogene Umsetzung in einem Gemisch von Dimethylacetamid und/oder N-Methylpyrrolidon mit LiCl nach Aktivierung des Celluloseausgangsproduktes ohne Anwesenheit von LiCl, deren Herstellung und deren Verwendung zu Membranen und Fasern beschrieben.

Die DE-A-38 05 992.4 betrifft modifizierte Cellulose für biocompatible Dialysemembranen, die eine durch die Formel

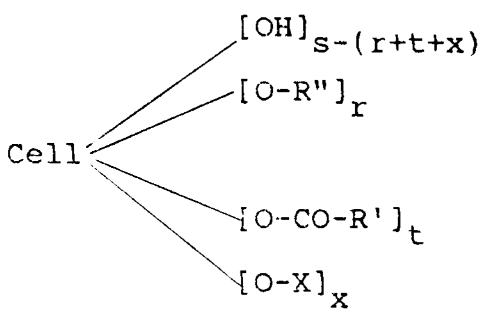

wiedergegebene Struktur aufweist, worin Cell Cellulose oder Chitin jeweils ohne Hydroxylgruppen ist, s bei Cellulose 3 und bei Chitin 2 beträgt und worin

**R'** : $CH_3$ und/oder $C_2H_5$ und/oder $C_3H_7$,
**X** : definierte funktionelle Gruppen bedeuten, und

wobei

**R''**: H oder R ist,
**x+t**: 1,00 - 2,50,
**x** : 0 - 0,5t,
**r** : 0,01 - 0,45 sind

und der Polymerisationsgrad (DP) 100 - 500 beträgt und ein Verfahren zur Herstellung der Cellulosederivate.

Die DE-A-38 05 966.5 betrifft modifizierte Cellulose für biocompatible Dialysemembranen, die eine durch die Formel

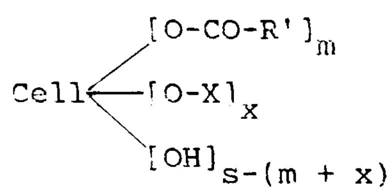

wiedergegebene Struktur aufweist, worin Cell unmodifizierte Cellulose oder Chitin jeweils ohne Hydroxylgruppen ist, s bei Cellulose 3 und bei Chitin 2 beträgt und
worin

**R'**: $CH_3$ und/oder $C_2H_5$ und/oder $C_3H_7$,
**X** : definierte funktionelle Gruppen bedeuten, und
**m** : 1,00 - 2,50,
**x** : 0,01 - 0,45 sind

und der Polymerisationsgrad (DP) 100 - 500 beträgt, und ein Verfahren zur Herstellung der modifizierten Cellulosederivate.

Die DE-A-38 05 973.8 betrifft modifizierte Cellulose für biocompatible Dialysemembranen, wobei die modifizierte Cellulose eine durch die Formel

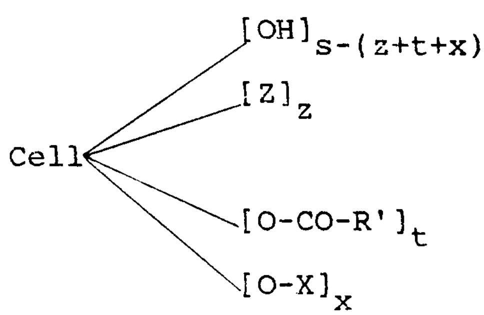

wiedergegebene Struktur aufweist, worin Cell Cellulose oder Chitin jeweils ohne Hydroxylgruppen ist, s bei Cellulose 3 und bei Chitin 2 beträgt und
worin

**R'** : $CH_3$ und/oder $C_2H_5$ und/oder $C_3H_7$,
**X** : definierte funktionelle Gruppen bedeuten, und
**Z** : den folgenden Atomgruppen entspricht:
SR'', $SO_3H$ (Salz), SO-R, SONR''$_2$, $SO_2$-R, $SO_2$NR''$_2$, $SO_2H$ (Salz), F, Cl, Br, J, NR''$_2$, PR''$_2$, $PO_3H_2$ (Salz), $PO_2H$(OR), PO(OR)$_2$, $PO_2$HR'' (Salz), POR''(OR), POR''$_2$

wobei

**R''**: H oder R ist,
**x+t**: 1,0 - 2,50,
**x** : 0 - 0,5t,
**z** : 0,01 - 0,45 sind

und der Polymerisationsgrad (DP) 100 - 500 beträgt, und ein Verfahren zur Herstellung der modifizierten Cellulose.

Neben dem Umstand, daß Dialysemembranen aus synthetischen bzw. natürlichen Polymeren bei ihrem Einsatz in künstlichen Nieren sehr leicht eine Gerinnung des Blutes hervorrufen können, die durch entsprechende medikamentöse Behandlung weitgehend verhindert wird, tritt bei Dialysemembranen aus regenerierter Cellulose häufig bei der Behandlung eines Nierenkranken mit Dialysatoren mit Cellulose-Membranen in der ersten Zeit der Dialysebehandlung ein vorübergehender Leukozytenabfall auf. Dieser Effekt wird als Leukopenie bezeichnet.

Leukopenie ist eine Erniedrigung der Leukozytenzahl (weiße Blutkörper) im Blutkreislauf. Die Zahl der weißen Blutkörper beim Menschen beträgt ca. 4000 bis 12000 Zellen/$mm^3$.

Die Leukopenie bei der Dialyse ist am stärksten ausgeprägt 15 bis 20 min. nach Beginn, wobei die Neutrophilen (das sind die mit neutralen oder gleichzeitig mit sauren und basischen Farbstoffen anfärbbaren

Leukozyten) fast vollständig verschwinden können. Danach erholt sich die Zahl der Leukozyten innerhalb etwa einer Stunde wieder auf fast den Ausgangswert oder übersteigt diesen.

Wird nach Erholung der Leukozyten ein neuer Dialysator angeschlossen, tritt wieder Leukopenie im gleichen Ausmaß ein.

Cellulose-Membranen verursachen eine ausgeprägte Leukopenie. Auch wenn die klinische Bedeutung der Leukopenie wissenschaftlich nicht geklärt ist, besteht doch der Wunsch nach einer Dialysemembran für die Hämodialyse, die den Effekt der Leukopenie nicht zeigt, ohne daß dadurch die anderen sehr erwünschten Eigenschaften von Dialysemembranen aus regenerierter Cellulose beeinträchtigt werden.

Bei der Hämodialyse mittels Membranen aus regenerierter Cellulose hat man neben der Leukopenie auch eine deutliche Komplement-Aktivierung festgestellt. Das Komplement-System innerhalb des Blutserums ist ein komplexes, aus vielen Komponenten bestehendes Plasmaenzym-System, das auf verschiedene Weise der Abwehr von Schädigungen durch eindringende fremde Zellen (Bakterien u.a.) dient. Wenn Antikörper gegen den eindringenden Organismus vorhanden sind, kann komplementspezifisch durch den Komplex der Antikörper mit antigenen Strukturen der Fremdzellen aktiviert werden, anderenfalls erfolgt auf einem Alternativ-Weg durch besondere Oberflächenmerkmale der Fremdzellen die Komplement-Aktivierung. Das Komplement-System beruht auf einer Vielzahl von Plasma-Proteinen. Nach Aktivierung reagieren diese Proteine spezifisch in einer bestimmten Reihenfolge miteinander und am Ende wird ein zellschädigender Komplex gebildet, der die Fremdzelle zerstört.

Aus einzelnen Komponenten werden Peptide freigesetzt, die Entzündungserscheinungen auslösen und gelegentlich auch unerwünschte pathologische Folgen für den Organismus haben können. Es wird angenommen, daß die Aktivierung bei Hämodialysemembranen aus regenerierter Cellulose über den alternativen Weg erfolgt. Objektiv festgestellt werden diese Komplement-Aktivierungen durch eine Bestimmung der Komplement-Fragmente C3a und C5a.

In diesem Zusammenhang wird auf folgende Arbeiten hingewiesen: D.E. Chenoweth et al., Kidney International Vol. 24, Seite 764 ff, 1983, und D.E. Chenoweth, Asaio-Journal Vol. 7, Seite 44 ff, 1984.

Das Karpal-Tunnel-Syndrom wird durch modifizierte Cellulosederivate beeinflußt. Es besteht aber ein erhebliches Bedürfnis nach weiteren Modifizierungen der Cellulose, um auch dieses Phänomen möglichst weitgehend auszuschalten.

Das Ziel der Erfindung war es, die Biokompatibilität von cellulosischen, d.h. von modifizierten und unmodifizierten Cellulosemembranen sowie Membranen auf Chitinbasis durch Nachbehandlung zu verbessern. Die Dialyse- und mechanischen Eigenschaften dürfen dabei nicht oder nur unwesentlich verschlechtert werden. Die Verknüpfung des Modifizierungsagens mit dem Polymer soll dabei nur über eine kovalente Bindung mit ausreichender Hydrolyse- und Lagerstabilität erfolgen, die auch eine Membransterilisation übersteht. Während der Blutdialyse darf kein Modifizierungsagens in die Blutbahn gelangen.

Gelöst wird diese Aufgabe durch ein Verfahren, das sich dadurch auszeichnet, daß man ein oder mehrere Lösungen die aus der Gruppe von von Säurechloriden, Säureanhydriden, Ketenen, Chlorkohlensäureestern, Isocyanaten, Thiocyanaten, Sulfonsäurechloriden, Sulfonsäureanhydriden, Phosphorsäureanhydrid, Ethylenoxid-, Ethylensulfid-, Ethylenimino-, Lacton-, Sulfon-, spaltbaren Onium-Verbindungen, Alkylaminoethanolschwefelsäureestern, Alkylsulfonethanol-schwefelsäureestern, Vinylsulfonsäure(Salz), Vinylsulfonsäureester, Vinylphosphonsäure(Salz), Vinylphosphonsäureester, Acrylamid, Methacrylamid, Acrylsäureester, Methacrylsäurester und/oder Acrylnitril und der Gruppe von polaren Lösungsmitteln ausgewählt werden, gegebenenfalls mit den zur Reaktion erforderlichen Katalysatoren und Hilfsstoffen, bei Raumtemperatur oder höheren Temperaturen an einer Oberfläche der Dialysemembran vorbeiführt, wobei die Dialysemembran durchgehend modifiziert wird und die so durchgehend modifizierte Dialysemembran mittels einer gründlichen Wäsche von den verbliebenen Modifizierungsagenzien und Nebenreaktionsprodukten befreit.

Die Nachbehandlung von cellulosischen Gebilden ist als solche nicht neu. Sie war besonders in den Jahren um 1960 Gegenstand von zahlreichen Arbeiten und Veröffentlichungen mit dem Ziel, cellulosischen Geweben bestimmte Eigenschaften wie Knittererholung, Hydrophobierung, Flammfestigkeit oder Antischmutzeigenschaften zu verleihen. Das Ausrüsten erfolgt üblicherweise durch Tränken des Cellulosegewebes mit der wäßrigen Lösung des Modifizierungsagens, Abpressen bis zur gewünschten Feuchtigkeitsaufnahme, Vortrocknen bei 100 °C und Fixieren bei 120 bis 150 °C. Danach werden die Gewebe gründlich gewaschen und getrocknet. In einigen Fällen werden auch als Ausrüstungsmedien organische Lösungsmittel verwendet. Versucht man nun, Dialysemembranen nach diesen Verfahrensweisen zu modifizieren, so stellt man fest, daß die Membranen ihre Dialyseeigenschaften weitgehend verlieren.

Die "Nachmodifizierung von Membranen zwecks Biokompatibilitätsverbesserung unter Beibehaltung der übrigen Membraneigenschaften" erfolgt beispielsweise erfindungsgemäß auf folgende allgemeine Weise:
Das Modifizierungsagens und ggf. die für die Reaktion erforderlichen Hilfsagenzien (Katalysator, säurebindendes Mittel) werden in einem organischen polaren Lösungsmittel gelöst, wobei Modifizierungs- und

Hilfsagens gemeinsam oder aus Verträglichkeits- bzw. Löslichkeitsgründen getrennt und auch in unterschiedlichen polaren Lösungsmitteln gelöst werden können. Die Modifizierung von Membranen erfolgt vom Modifizierungsagens abhängig entweder bei Raumtemperatur oder bei höheren Temperaturen. Nach der Modifizierung werden die Membranen mit Waschmitteln, in denen sowohl die Modifizierungsagenzien als auch die bei der Reaktion evtl. entstehenden Nebenreaktionsprodukte löslich sind, gründlich gewaschen. Im Falle, daß das Waschmittel einen hohen Siedepunkt hat oder Wasser ist, wird es durch ein niedrig siedendes organisches Lösungsmittel wie Alkohol, Aceton u. a. ausgetauscht, das ggf. auch ein Weichmachungsmittel enthalten kann. Die Membranen werden dann mit Luft oder Stickstoff bei 20 bis 70 °C getrocknet. Nach dieser Verfahrensweise werden biokompatible Dialysemembranen erhalten, die nicht nur oberflächlich sondern durchgehend modifiziert sind und weitgehend die gleichen Dialyseeigenschaften wie vor der Modifizierung zeigen.

Als polare Lösungsmittel sind Alkohole mit 1 - 8 C-Atomen, Ether, Ketone, amidische Lösungsmittel, Nitromethan, Acetonitril, Dimethylsulfoxid, Halogenkohlenwasserstoffe und/oder tertiäre Aminogruppen enthaltende Lösungsmittel geeignet.

Als Lösungsmittel aus der Gruppe der Sauerstoff enthaltenden Lösungsmittel kommen beispielsweise Ether und Ketone in Frage, als solche oder im Gemisch mit anderen polaren Lösungsmitteln, beispielsweise Alkohole mit 1 - 4 C-Atomen, Glykole wie Neopentylglykol, Triethylenglykol, Polyethylenglykole, Cyclohexanole wie Cyclohexanol, Methylcyclohexanol, Benzylalkohol, Furfurylalkohol,
Ethylglykol, Propylglykol, Butylglykol, Hexyldiglykol, Diglykoldimethylether, Propylenglykoldimethylether, Tripropylenglykolmethylether, Triethylenglykoldimethylether, Hexylglykol, Methyldiglykol, Ethyldiglykol, Butyldiglykol, Methyltriglykol, Ethyltriglykol, Butyltriglykol, Ethylenglykoldimethylether, Triethylenglykoldimethylether, Diethylenglykoldiethylether,
Methylpropylketon, Methylisopropylketon, Methylbutylketon, Methylisobutylketon, Methylamylketon, Methylisoamylketon, Methylheptylketon, Diethylketon, Ethylbutylketon, Ethylamylketon, Diisopropylketon, Diisobutylketon, Methylcyclohexanon, 3-Methylcycloheptanon-5, Mesityloxid, Acetylaceton, Isopropylacetat, Butylacetat, Isobutylacetat, sec-Butylacetat, Amylacetat, Isoamylacetat, Hexylacetat, Cyclohexylacetat, Benzylacetat, Methylpropionat, Ethylpropionat, Propylpropionat, n-Butylpropionat, Ethylbutyrat, Propylbutyrat, n-Butylbutyrat, Isobutylbutyrat, Amylbutyrat, Methylisobutyrat, Ethylisobutyrat, Isopropylisobutyrat, Isobutylisobutyrat, Ethylacetat, Butylacetat, Glykolsäurebutylester, Methylglykolacetat, Ethylglykolacetat, Butylglykolacetat, Ethyldiglykolacetat, Butyldiglykolacetat, 3-Methoxybutylacetat, Ethylencarbonat und Propylencarbonat.
Bevorzugt sind aus diesen Lösungsmittelgruppen jedoch Wasser, Alkohole mit 1 - 4 C-Atomen, Ethylenglykol, Diethylenglykol, Propandiol, Butandiol, Glycerin, Dioxan, Methylglykol, Ethylglykol, Aceton, Methylethylketon, Cyclohexanon, Methylacetat, Ethylacetat, Propylacetat, Ethylencarbonat, Propylencarbonat, gegebenenfalls im Gemisch mit anderen polaren Lösungsmitteln.

Weitere geeignete Lösungsmittel sind beipielsweise amidische Lösungsmittel, wie Dimethylformamid, Dimethylacetamid und N-Methylpyrrolidon als solche oder im Gemisch mit anderen polaren Lösungsmitteln.

Geeignet sind beispielsweise auch chlorierte Kohlenwasserstoffe wie 1.1-Dichlorethan, 2-Chlorbutan, 1.1.1-Trichlorethan, Tetrachlorkohlenstoff, 1-Chlorbutan, 1.2-Dichlorethan, Trichlorethylen, Perchlorethylen, 1.1.2.2-Tetrachlorethan, Dichlordiethylether, o-Dichlorbenzol und o-Chlortoluol.

Bevorzugt sind dabei Chlorpropan, Methylenchlorid, Chloroform oder Chlorbenzol, gegebenenfalls im Gemisch mit anderen polaren Lösungsmitteln.

Gegenstand der Erfindung ist auch eine Vorrichtung zur Durchführung des Verfahrens. Sie besteht aus einem Rohrleitungssytem (14), das mit einem oder mehreren Vorratsgefäßen für Flüssigkeiten (1, 2, 3, 4) und/oder Gasen (6) und mit einem oder mehreren Membranmodulen (13) verbunden ist und einer Fördereinrichtung (16) zur Aufrechterhaltung eines Flüssigkeitsumlaufes, wobei das Rohrleitungssystem (14) eine Ringleitung ist, an die nacheinander über Dosiereinrichtungen (17) die Vorratsgefäße (1, 2, 3, 4, 6) angeschlossen sind, und daß die Dosiereinrichtungen (17) derart ausgebildet sind, daß die Reaktanten, Lösungsmittel und/oder Waschmittel zu vorgegebenen Zeiten und in vorgegebener Menge über die Ringleitung wenigstens einem Kompartiment der Membranmodule (13) zuführbar sind.

Unter Membranmodulen sollen im Rahmen der Erfindung im weitesten Sinne Einheiten mit Membranen in Form von Flachfolien, Schlauchfolien oder Hohlfäden verstanden werden. Sie können in ein mit Anschlußstücken versehenes Gehäuse fest eingebaut oder in anderer Weise mit oder ohne Dichtmittel zusammengefaßt sein, beispielsweise zu einem Hohlfadenbündel mit einer schlauchförmigen Umhüllung.

Vorzugsweise sind mehrere Membranmodule in einen mit Durchflußöffnungen versehenen Aufnahmebehälter (12) und ein oder mehrere Aufnahmebehälter (12) in ein Reaktionsgefäß (9) eingebracht, welches in die Ringleitung (14) zwischengeschaltet ist.

Dabei sind die Membranmodule in möglichst dichter Packung in dem Aufnahmebehälter (12) angeordnet.

Zur nachträglichen Modifizierung von Membranmodulen einschließlich Hohlfadenbündeln und Flachmembranen sind die in der Figur 1 bzw. 2 schematisch dargestellten Apparaturen sehr gut geeignet.

In Figur 1 sind mehrere Membranmodule (13) unmittelbar mit der Ringleitung des Rohrleitungssystems (14) verbunden. Im einzelnen bedeuten dabei (1) Vorratsgefäß für beispielswelse Reaktant-Lösung, (2) Vorratsgefäß für beispielsweise Hilfsagens-Lösung (Katalysator, Säurebindemittel), (3) Vorratsgefäß für beispielsweise Lösungsmittel, (4) Vorratsgefäß für beispielsweise Waschlösung mit Weichmacher, (5) Druckmeß- und -regelung, (6) Vorratsgefäß für Gase, (7) Kondensator, (8) Entlüftung, (10) Temperaturmeß- und regelung, (11) Entleerung, (13) Membranmodul, (14) Rohrleitungssystem, (15) Durchflußmeß- und -regelung, (16) Fördereinrichtung, (17) Dosiereinrichtung, (18) Temperaturmeß- und registrierung, (19) Durchflußmeß- und -registrierung.

In Figur 2 wird die bevorzugte Ausführungsform gemäß Patentanspruch 13 dargestellt, in der (12) den Aufnahmebehälter und (9) das Reaktionsgefäß bedeuten. Die übrigen Bezugsziffern haben die gleiche Bedeutung wie in Figur 1.

Figur 3 und 4 sind Schnittaufnahmen von erfindungsgemäßen Membranen, die mit einem basischen Farbstoff, der unmodifizierte Cellulose nicht färbt, gefärbt wurden. Es ist deutlich sichtbar, daß die Färbung im gesamten Querschnitt gleichmäßig ist, was bestätigt, daß die Modifizierung über den gesamten Fadenquerschnitt erfolgt, obwohl die Reaktanten nur mit der Oberfläche der Cellulose in Berührung standen.

In Figur 5 ist das IR-Spektrum der erfindungsgemäßen modifizierten Cellulosemembran gemäß Beispiel 24 dargestellt.

Im Rahmen der vorliegenden Erfindung wurde die Komplement-Aktivierung anhand der Fragmente C5a beurteilt. Dazu wurden in vitro 300 ml heparinisiertes Blutplasma über einen Zeitraum von 4 Std. mit einem Plasmafluß von 100 ml/min durch einen Dialysator mit 1 $m^2$ effektiver Austauschfläche rezirkuliert. In dem Plasma wurden die C5a-Fragmente mit Hilfe der RIA-Methode (Upjohn-Test) bestimmt. Die relative Komplement-Aktivierung für den jeweiligen Meßzeitpunkt wurde durch Bildung des Verhältnisses aus der Konzentration zum Zeitpunkt der Probenahme und dem Anfangswert in Prozent errechnet. Zur Bewertung wurde der Meßwert nach 4 Std. Rezirkulationszeit herangezogen. Flachmembranen werden mit heparinisiertem Blutplasma 3 Stunden inkubiert und anschließend die C5a-Fragmente bestimmt.

Die Erhöhung des beta-2-Mikroglobulinspiegels bei Langzeit-Dialysepatienten wird nach Verwendung von Membranen aus regenerierter Cellulose beobachtet und wird darauf zurückgeführt, daß diese Membranen im Molekularbereich von 1000 bis 20.000 weniger durchlässig sind und die Mikroglobuline bei der Dialyse deshalb nicht in ausreichendem Maße entfernt werden. An die üblichen Membranen aus regenerierter Cellulose adsorbiert sich das beta-2-Mikroglobulin nicht in nennenswertem Umfang. Hierzu aber können in unerwarteter Weise die erfindungsgemäßen Cellulosederivate beitragen.

Gemessen wird im Rahmen der Erfindung der beta-2-Mikroglobulingehalt, der an die Membran adsorbiert wird, auf folgende Weise.

In je 500 mg Substanz (Dialysemembran) werden 10 ml Humanblutplasma gegeben und 30 min bei 37 °C inkubiert. Das Humanblutplasma hat einen Gehalt an beta-2-Mikroglobulin von 13,67 mg/l. Die Probe wird bei 3000 Upm 15 min zentrifugiert. Im Überstand wird der Gehalt an beta-2-Mikroglobulin festgestellt. Anschließend wird die Probe 2 x mit je 10 ml Phosphat-buffer-saline gewaschen. In den Waschflüssigkeiten wird der Mikroglobulingehalt ebenfalls festgestellt. Aus der Differenz zwischen ursprünglichem und nicht absorbiertem beta-2-Mikroglobulin läßt sich die prozentuale Menge an absorbiertem beta-2-Mikroglobulin errechnen.

Der Durchschnittspolymerisationsgrad DP wurde in einer Cuen-Lösung nach DIN 54270 bestimmt.
Der Veretherungsgrad und/oder Veresterungsgrad wurden anhand der Analysenergebnisse bestimmt, die für die Substituenten bekannt und typisch sind, beispielsweise Stickstoff nach Kjeldahl, Schwefel nach Schöniger oder Phosphor nach der Molybdatmethode, gegebenenfalls aus der Differenz vor und nach einer Verseifung.

Zur Veresterung können die bekannten Katalysatoren wie Basen, Säuren, basische Salze u. a. angewandt werden. Wegen der Gefahr eines Abbaues der Cellulose kommen jedoch vorzugsweise Basen und basische Salze in Frage.

Bei der Umsetzung mit Isocyanaten können ebenfalls bekannte Katalysatoren verwendet werden. Aus Abbaugründen werden vorzugsweise tertiäre Basen verwendet.

Bei der Veretherung werden ebenfalls bekannte Agenzien eingesetzt.

Anhand der nachfolgenden Beispiele wird die Erfindung näher erläutert.

Beispiel 1

486 g (3 Mol) Cellulosehohlfäden wurden in einem Reaktor entsprechend der Figur 2 vorgelegt. Die Apparatur wurde mit einer Lösung bestehend aus 3 l Dimethylacetamid, 60 g (0,45 Mol) p-Tolylisocyanat und 5 ml Triethylamin vollgefüllt, wobei die Luft aus den Hohlfäden und der Anlage durch Absaugen vollständig entfernt wurde. Der Reaktor wurde auf 50 °C aufgeheizt und die Lösung mit Hilfe einer Pumpe während 24 Stunden umgewälzt. Danach wurde die das Modifizierungsagens enthaltende Lösung aus dem Reaktor abgelassen, die Hohlfäden wurden mit Dimethylacetamid, Ethanol und schließlich mit 1 %iger alkoholischer Glycerinlösung gespült und mit Stickstoff im Reaktor bei 50 °C getrocknet. Die auf diese Weise behandelten Cellulosehohlfäden weisen einen Stickstoffgehalt von 0,8 %, entsprechend einem Substitutionsgrad von m = 0,10, auf.

Der UFR-Wert bleibt mit 4 ml/h.m$^2$.mm Hg bei 37 °C unverändert.

Im Vergleich zu unmodifizierten Hohlfäden zeigen die modifizierten Hohlfäden eine geringere Komplementaktivierung. Gegenüber der unmodifizierten Membran beträgt die C5a-Reduktion 100 %.

Beispiele 2 - 8

Auf der Grundlage der Arbeitsweise von Beispiel 1 wurden mit einer Anzahl von Isocyanaten in Dimethylacetamid und literaturbekannten Katalysatoren Cuprophan- und Chitinhohlfäden nachbehandelt und deren Komplementaktivierung anhand der Fragmente C5a bestimmt. Die Ergebnisse sind in der Tabelle 1 zusammengestellt.

Beispiel 9

16,2 g (0,1 Mol) Cuprophanflachmembranen wurden in einem zylindrischen Gefäß vorgelegt. Dann wurde eine Lösung bestehend aus 800 ml Dimethylacetamid, 26,6 g (0,1 Mol) Dodecenylsuccinat und 2,5 g (0,025 Mol) Kaliumacetat zugefügt und die Reaktionsmischung 10 Stunden bei 60 °C und 15 Stunden bei 20 °C gehalten. Anschließend wurden die Membranen mit Dimethylacetamid, Ethanol, Wasser, Ethanol und 1%iger alkoholischer Glycerinlösung gewaschen und an der Luft getrocknet. Die Membranen weisen einen Dodecenylgruppengehalt von m = 0,13 auf. Der UFR-Wert bleibt mit 2,4 ml/h.m$^2$.mm Hg bei 37 °C unverändert.

Gegenüber der unmodifizierten Membran beträgt die C5a-Reduktion 100 %. Außerdem zeigt die Membran eine Beta-2-Microglobulin-Adsorption von 38 %.

Beispiel 10 - 18

Auf der Grundlage der Arbeitsweise von Beispiel 9 wurden Cuprophan- und Chitinflachmembranen durch Veresterung nachmodifiziert und untersucht. Die Ergebnisse sind in der Tabelle 2 zusammengestellt.

Beispiel 19

486 g (3 Mol) Cellulosehohlfäden wurden in einem Reaktor entsprechend der Figur 2 vorgelegt. Die Apparatur wurde mit einer Lösung bestehend aus 151,5 g (1 Mol) 2,3-Epoxypropyltrimethylammoniumchlorid, 18 g (0,45 Mol) Natriumhydroxid und 2850 g Wasser vollgefüllt, wobei die Luft aus den Hohlfäden und der Anlage durch Absaugen vollständig entfernt wurde. Die Reaktionslösung wurde mit Hilfe einer Pumpe während 24 Stunden bei 25 °C umgewälzt, dann abgelassen, die Membranen wurden mit Wasser, Ethanol und 1 %iger alkoholischer Glycerinlösung gespült und mit Stickstoff im Reaktor bei 50 °C getrocknet. Die Membranen weisen einen Stickstoffgehalt von 0,17 %, entsprechend einem Substitutionsgrad von m = 0,02, auf.

Gegenüber den unmodifizierten Hohlfäden beträgt die C5a-Reduktion 78 %.

Beispiel 20

Auf der Grundlage der Arbeitsweise von Beispiel 19 wurden Cellulosehohlfäden mit Vinylsulfonsäure-Natriumsalz und Natriumhydroxid als Katalysator nachmodifiziert. Die Membranen weisen einen Modifizierungsgrad von m = 0,05 auf.

Durch Anfärbung mit einem basischen Farbstoff, beispielsweise aus der Astrazonblau-Serie, der nur auf saure Gruppen anspricht und unmodifizierte Cellulose nicht anfärbt, sieht man deutlich, daß die modifizierten Hohlfäden durchgehend gleichmäßig angefärbt sind (Fig. 3 und 4). Dies deutet indirekt auf eine durchgehende Modifizierung hin.

Gegenüber der unmodifizierten Membran beträgt die C5a-Reduktion 70 %.

Beispiel 21

486 g (3 Mol) Cellulosehohlfäden wurden in einem Reaktor entsprechend der Figur 2 vorgelegt. Die Membranen wurden mit 3 %iger wäßriger Natriumhydroxid-Lösung 1/2 Stunden bei 15 °C alkalisiert und mit Aceton gespült. Die Apparatur wurde mit einer Lösung bestehend aus 81,3 g (0,6 Mol) Chlorethyldiethylamin und 2950 ml i-Propanol vollgefüllt, wobei die Luft aus den Hohlfäden und der Anlage durch Absaugen vollständig entfernt wurde. Der Reaktor wurde auf 60 °C erhitzt und die Lösung 2 Stunden mit Hilfe einer Pumpe umgewälzt. Nach dem Ablassen der Reaktionslösung wurden die Membranen mit Wasser, Ethanol und 1%iger alkoholischer Glycerinlösung gespült und mit Stickstoff im Reaktor bei 50 °C getrocknet. Die Membranen weisen einen Stickstoffgehalt von 0,21 %, entsprechend einem Substitutionsgrad von m = 0,025, auf.

Gegenüber der unmodifizierten Membran beträgt die C5a-Reduktion 85 %.

Beispiel 22

Auf der Grundlage der Arbeitsweise von Beispiel 21 wurden Cellulosehohlfäden mit Acrylnitril in 1,2-Dichlorethan und Natriumhydroxid als Katalysator nachmodifiziert. Die Membranen weisen einen Modifizierungsgrad von m = 0,08 auf.

Gegenüber der unmodifizierten Membran beträgt die C5a-Reduktion 94 %.

Beispiel 23

Auf der Grundlage der Arbeitsweise von Beispiel 21 wurden Cellulosehohlfäden mit Ethyleniminbernsteinsäurediethylester in Dimethylacetamid und Methansulfonsäure als Katalysator nachbehandelt. Dabei resultierten Membranen mit einem Substitutionsgrad von m = 0,023.

Gegenüber der unmodifizierten Membran beträgt die C5a-Reduktion 68 %.

Beispiel 24

32,4 g (0,2 Mol) Cellulosehohlfäden wurden zu einem Modul verarbeitet und an eine Apparatur entsprechend der Figur 1 angeschlossen. Die Apparatur wurde mit einer Lösung bestehend aus 8 g (0,06 Mol) p-Tolylisocyanat, 1 ml Triethylamin und 500 ml Dimethylacetamid vollgefüllt, wobei die Luft durch Absaugen vollständig entfernt wurde. Die Membranen wurden zunächst 7 Stunden bei 50 °C und dann 15 Stunden bei 25 °C mit der Reaktionslösung behandelt. Dabei wurde die Lösung mit Hilfe einer Pumpe ständig umgewälzt. Nach der Entfernung der Lösung wurden die Membranen mit Dimethylacetamid, Ethanol und 1 %iger alkoholischer Glycerinlösung gespült und mit Stickstoff bei 50 °C getrocknet. Die resultierenden Membranen enthalten 0,45 % Stickstoff, entsprechend einem Substitutionsgrad von m = 0,06.

Das IR-Spektrum (Figur 5) zeigt bei 1719, 1531 und 1602 $cm^{-1}$ die für ein aromatisches Urethan charakteristischen Banden.

Gegenüber der unmodifizierten Membran beträgt die C5a-Reduktion 98 %.

Beispiele 25 - 28

Auf der Grundlage der Arbeitsweise von Beispiel 1 oder 9 wurden Chitosanmembranen mit Isocyanaten oder durch Veresterung nachmodifiziert und untersucht. Die Ergebnisse sind in der Tabelle 3 zusammengestellt.

Tabelle 1

Allgemeine Formel: 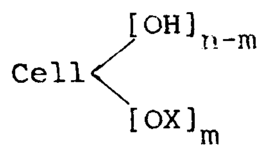 (Cell = Cellulose- bzw. Chitinmolekül ohne OH-Gruppen)

| Beispiel Nr. | Polymer | n | X | m | C5a-Redukt. % | Beta-2-Microglob. Adsorpt. % |
|---|---|---|---|---|---|---|
| 2 | Cellulose | 3 | $C_4H_9NHCO$ | 0,15 | 94 | - |
| 3 | Cellulose | 3 | $C_6H_{11}$-NHCO | 0,11 | 99 | 10 |
| 4 | Cellulose | 3 | $C_6H_5$-NHCO | 0,13 | 100 | - |
| 5 | Cellulose | 3 | $C_{18}H_{37}$-NHCO | 0,05 | 93 | 12 |
| 6 | Cellulose | 3 | OCNH-$C_6H_4$-NHCO | 0,06 | 82 | - |
| 7 | Cellulose | 3 | OCNH-$C_6H_{10}$-NHCO | 0,07 | 86 | 8 |
| 8 | Chitin | 2 | $CH_3$-$C_6H_4$-NHCO | 0,12 | 97 | - |

Tabelle 2

Allgemeine Formel: 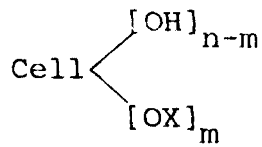 (Cell = Cellulose- bzw. Chitinmolekül ohne OH-Gruppen)

| Beispiel Nr. | Polymer | n | X | m | C5a-Redukt. % | Beta-2-Microglob Adsorpt. % |
|---|---|---|---|---|---|---|
| 10 | Cellulose | 3 | $C_2H_5CO$ | 0,55 | 99 | - |
| 11 | Cellulose | 3 | $C_3H_7CO$ | 0,28 | 96 | - |
| 12 | Cellulose | 3 | $C_{17}H_{35}CO$ | 0,05 | 93 | - |
| 13 | Cellulose | 3 | $C_{15}H_{31}CH{=}C(CH_2COOH)CO$ | 0,09 | 100 | 33 |
| 14 | Cellulose | 3 | $C_{11}H_{23}COCH(C_{10}H_{21})CO$ | 0,08 | 95 | - |
| 15 | Cellulose | 3 | $C_{17}H_{35}COCH(C_{16}H_{33})CO$ | 0,05 | 100 | - |
| 16 | Cellulose | 3 | $HOOCCH(SO_3H)CH_2CO$ | 0,07 | 96 | 43 |
| 17 | Cellulose | 3 | $C_2H_5CO/HOOCCH{=}CHCO$ | 0,35/ 0,08 | 97 | - 27 |
| 18 | Chitin | 2 | $C_{11}H_{23}CH{=}C(CH_2COOH)CO$ | 0,06 | 85 | 25 |

Tabelle 3

Allgemeine Formel: 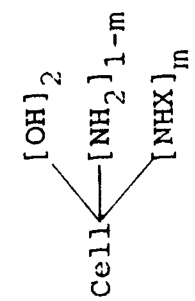

(Cell = Cellulosemolekül ohne OH-Gruppen)

| Beispiel Nr. | Polymer | X | m | C5a-Redukt. % | Beta-2-Microglob. Adsorpt. % |
|---|---|---|---|---|---|
| 25 | Chitosan | $CH_3-C_4H_6-NHCO$ | 0,18 | 90 | - |
| 26 | Chitosan | $C_{18}H_{37}-NHCO$ | 0,07 | 85 | - |
| 27 | Chitosan | $C_{11}H_{23}CH{=}C(CH_2COOH)CO$ | 0,10 | 93 | 24 |
| 28 | Chitosan | $C_{15}H_{31}CH{=}C(CH_2COOH)CO$ | 0,09 | 95 | 28 |

## Patentansprüche

1. Verfahren zur chemischen Modifizierung von cellulosischen Dialysemembranen sowie solchen auf Chitinbasis in Form von Flachfolien, Schlauchfolien oder Hohlfäden zur Verbesserung der Biokompatibilität, ausgedrückt durch eine Reduzierung der C5a-Komplementaktivierung und einer erhöhten $\beta$2-Microglobulin-Adsorption, dadurch gekennzeichnet, daß man eine oder mehrere Lösungen, die aus der Gruppe von Säurechloriden, Säureanhydriden, Ketenen, Chlorkohlensäureestern, Isocyanaten, Thiocyanaten, Sulfonsäurechloriden, Sulfonsäureanhydriden, Phosphorsäureanhydrid, Phosphonsäurechloriden, Ethylenoxid-, Ethylensulfid-, Ethylenimino-, Lacton-, Sulfon-, spaltbaren Onium-Verbindungen,

Alkylaminoethanolschwefelsäureestern, Alkylsulfonethanolschwefelsäureestern, Vinylsulfonsäure(Salz), Vinylsulfonsäureester, Vinylphosphonsäure(Salz), Vinylphosphonsäureester, Acrylamid, Methacrylamid, Acrylsäureester, Methacrylsäureester und/oder Acrylnitril und der Gruppe von polaren Lösungsmitteln ausgewählt werden, gegebenenfalls mit den zur Reaktion erforderlichen Katalysatoren und Hilfsstoffen, bei Raumtemperatur oder höheren Temperaturen an einer Oberfläche der Dialysemembran vorbeiführt, wobei die Dialysemembran durchgehend modifiziert wird und die so durchgehend modifizierte Dialysemembranen mittels einer gründlichen Wäsche von den verbliebenen Modifizierungsagenzien und Nebenreaktionsprodukten befreit werden.

**2.** Verfahren nach Anspruch 1, dadurch gekennzeichnet, daß als polare Lösungsmittel Alkohole mit 1 - 8 C-Atomen, Ether, Ketone, amidische Lösungsmittel, Nitromethan, Acetonitril, Dimethylsulfoxid, Halogenkohlenwasserstoffe oder tertiäre Aminogruppen enthaltende Lösungsmittel eingesetzt werden.

**3.** Verfahren nach Anspruch 1 oder 2, dadurch gekennzeichnet, daß als Lösungsmittel Wasser, gegebenenfalls im Gemisch mit anderen polaren Lösungsmitteln, eingesetzt wird.

**4.** Verfahren nach Anspruch 1 oder 2, dadurch gekennzeichnet, daß als Lösungsmittel aus der Gruppe der Sauerstoff enthaltenden Lösungsmittel Ether und/oder Ketone als solche oder im Gemisch mit anderen polaren Lösungsmitteln, Alkohole mit 1 - 4 C-Atomen, Ethylenglykol, Propandiol, Butandiol, Dioxan, Methylglykol, Ethylglykol, Aceton, Methylethylketon oder Cyclohexanon, Ethylacetat, Ethylencarbonat eingesetzt werden.

**5.** Verfahren nach Anspruch 1 oder 2, dadurch gekennzeichnet, daß als amidische Lösungsmittel Dimethylacetamid, Dimethylformamid oder N-Methylpyrrolidon als solche oder im Gemisch mit anderen polaren Lösungsmitteln eingesetzt werden.

**6.** Verfahren nach Anspruch 1 oder 3, dadurch gekennzeichnet, daß als Lösungsmittel Chlorpropan, Methylenchlorid, Chloroform oder o-Chlortoluol als solche oder im Gemisch mit anderen polaren Lösungsmitteln eingesetzt werden.

**7.** Verfahren nach einem oder mehreren der Ansprüche 1 bis 6, dadurch gekennzeichnet, daß der Modifizierung der Dialysemembran eine Behandlung mit einem polaren Lösungsmittel vorausgeht.

**8.** Verfahren nach Anspruch 7, dadurch gekennzeichnet, daß aus einer Hohlfadenmembran mit dem polaren Lösungsmittel gegebenenfalls vorhandene Restmengen an hohlraumbildender Flüssigkeit gleichzeitig ausgewaschen werden.

**9.** Vorrichtung zur Durchführung des Verfahrens bestehend aus einem Rohrleitungssytem (14), das mit einem oder mehreren Vorratsgefäßen für Flüssigkeiten (1, 2, 3, 4) und/oder Gasen (6) und mit einem oder mehreren Membranmodulen (13) verbunden ist und einer Fördereinrichtung (16) zur Aufrechterhaltung eines Flüssigkeitsumlaufes, dadurch gekennzeichnet, daß das Rohrleitungssystem (14) eine Ringleitung ist, an die nacheinander über Dosiereinrichtungen (17) die Vorratsgefäße (1, 2, 3, 4, 6) angeschlossen sind und daß die Dosiereinrichtungen (17) derart ausgebildet sind, daß die Reaktanten, Lösungsmittel und/oder Waschmittel zu vorgegebenen Zeiten und in vorgegebener Menge über die Ringleitung wenigstens einem Kompartiment der Membranmodule (13) zuführbar sind.

**10.** Vorrichtung nach Anspruch 9, dadurch gekennzeichnet, daß mehrere Membranmodule in einen mit Durchflußöffnungen versehenen Aufnahmebehälter (12) und ein oder mehrere Aufnahmebehälter (12) in ein Reaktionsgefäß (9) eingebracht sind, welches in die Ringleitung (14) zwischengeschaltet ist.

**11.** Vorrichtung nach Anspruch 10, dadurch gekennzeichnet, daß die Membranmodule in möglichst dichter Packung in dem Aufnahmebehälter (12) angeordnet sind.

**Claims**

**1.** A process for the chemical modification of cellulosic dialysis membranes and of those on a chitin basis in the form of flat films, tubular films or hollow threads for improving the biocompatibility as expressed by a reduction in the C5a complement activation and increased β2-microglobulin adsorption, characterised in that one or more solutions selected from acid chlorides, acid anhydrides, ketenes, chlorocarbonic acid esters, isocyanates, thiocyanates, sulphonic acid chlorides, sulphonic acid anhydrides, phosphoric acid anhydride, phosphonic acid chlorides, ethylene oxide compounds, ethylene sulphide compounds, ethyleneimino compounds, lactone compounds, sulphone compounds, decomposable onium compounds, alkylamino-ethanol sulphuric acid esters, alkyl sulphonoethanol sulphuric acid esters, vinyl sulphonic acid (salt), vinyl sulphonic acid esters, vinyl phosphonic acid (salt), vinyl phosphonic acid esters, acrylamide, methacrylamide, acrylic acid esters, methacrylic acid esters and/or acrylonitrile and from polar solvents is or are moved past a surface of the dialysis membrane at room temperature or elevated temperatures, optionally together with the catalysts required for the reaction and auxiliary substances, the dialysis membrane being modified throughout and the thus completely modified dialysis membranes being freed from the remaining modifying agents and side reaction products by a thorough washing.

**2.** A process according to Claim 1, characterised in that the polar solvents used are alcohols having 1 - 8 carbon atoms, ethers, ketones, amidic solvents, nitromethane, acetonitrile, dimethylsulphoxide, halogenated hydrocarbons or solvents containing tertiary amino groups.

**3.** A process according to Claim 1 or Claim 2, characterised in that the solvent used is water optionally mixed with other polar solvents.

**4.** A process according to Claim 1 or Claim 2, characterised in that the solvents used are selected from oxygen-containing solvents, ethers and/or ketones as such or in admixture with other polar solvents, alcohols having 1 to 4 carbon atoms, ethylene glycol, propanediol, butanediol, dioxane, methyl glycol, ethyl glycol, acetone, methyl ethyl ketone or cyclohexanone, ethyl acetate, ethylene carbonate.

**5.** A process according to Claim 1 or Claim 2, characterised in that the amidic solvents used are dimethylacetamide, dimethylformamide or N-methylpyrrolidone, either as such or in admixture with other polar solvents.

**6.** A process according to Claim 1 or Claim 3, characterised in that the solvents used are chloropropane, methylene chloride, chloroform or o-chlorotoluene, either as such or in admixture with other polar solvents.

**7.** A process according to one or more of Claims 1 to 6, characterised in that modification of the dialysis membrane is preceded by a treatment with a polar solvent.

**8.** A process according to Claim 7, characterised in that any residues of cavity forming liquid are washed out of a hollow thread membrane with the polar solvent at the same time.

**9.** Apparatus for carrying out the process, consisting of a system of pipes (14) which is connected with one or more storage vessels for liquids (1, 2, 3, 4) and/or gases (6) and with one or more membrane modules (13), and a conveyor device (16) for maintaining a liquid circulation, characterised in that the pipe system (14) is a closed circular pipeline to which the storage vessels (1, 2, 3, 4, 6) are connected in succession by way of dosing devices (17) and in that the dosing devices (17) are so constructed that the reactants, solvents and/or washing fluids can be delivered at the predetermined times and in predetermined quantities to at least one compartment of the membrane module (13) by way of the circular pipeline.

**10.** Apparatus according to Claim 9, characterised in that several membrane modules are introduced into a receiver (12) provided with throughflow openings and one or more receivers (12) are introduced into a reaction vessel (9) which is interposed in the circular pipeline (14).

**11.** Apparatus according to Claim 10, characterised in that the membrane modules are arranged as densely packed as possible in the receiver (12).

## Revendications

1. Procédé de modification chimique de membranes de dialyse cellulosiques et de membranes de dialyse à base de chitine, sous la forme de feuilles planes, de feuilles tubulaires ou de fibres creuses, pour l'amélioration de la biocompatibilité, exprimée par une réduction de l'activation du complément C5a et une adsorption accrue de la $\beta$2-microglobuline, **caractérisé** en ce que l'on fait passer sur une surface de la membrane de dialyse, à la température ambiante ou à une température plus élevée, éventuellement avec les catalyseurs et produits auxiliaires nécessaires à la réaction, une ou plusieurs solutions qui sont choisies dans le groupe constitué des chlorures d'acide, anhydrides d'acide, cétènes, esters de l'acide chlorocarbonique, isocyanates, thiocyanates, chlorures d'acide sulfonique, anhydrides d'acide sulfonique, anhydride d'acide phosphorique, chlorures d'acide phosphonique, composés du type oxyde d'éthylène, composés du type sulfure d'éthylène, composés du type éthylèneimino, composés du type lactone, composés du type sulfone, composés onium scindables, esters d'acide alkylaminoéthanol sulfurique, esters d'acide alkylsulfonyléthanolsulfurique, acide vinylsulfonique (sels), esters d'acide vinylsulfonique, acide vinylphosphonique (sels), esters d'acide vinylphosphonique, acrylamide, méthacrylamide, esters d'acide acrylique, esters d'acide méthacrylique et acrylonitrile, et dans le groupe des solvants polaires, ce qui produit une modification continue de la membrane de dialyse, et on débarrasse, au moyen d'un lavage prolongé, les membranes de dialyse ainsi modifiées de manière continue des agents de modification et produits de réaction secondaire restants.

2. Procédé selon la revendication 1, **caractérisé** en ce que l'on utilise, comme solvants polaires, des alcools ayant 1 à 8 atomes de carbone, des éthers, des cétones, des solvants du type amide, le nitrométhane, l'acétonitrile, le diméthylsulfoxyde, des hydrocarbures halogénés ou des solvants renfermant des groupes amino-tertiaires.

3. Procédé selon la revendication 1 ou 2, **caractérisé** en ce que l'on utilise, comme solvant, de l'eau, éventuellement en mélange avec d'autres solvants polaires.

4. Procédés selon la revendication 1 ou 2, **caractérisé** en ce que l'on utilise, comme solvants pris dans le groupe des solvants renfermant de l'oxygène, des éthers et/ou des cétones, tels quels ou en mélange avec d'autres solvants polaires, des alcools ayant 1 à 4 atomes de carbone, l'éthylèneglycol, le propanediol, le butanediol, le dioxane, le méthylglycol, l'éthylglycol, l'acétone, la méthyléthylcétone, la cyclohexanone, l'acétate d'éthyle, le carbonate d'éthylène.

5. Procédé selon la revendication 1 ou 2, **caractérisé** en ce que l'on utilise, comme solvants du type amide, le diméthylacétamide, le diméthylformamide ou la N-méthylpyrrolidone, tel quel ou en mélange avec d'autres solvants polaires.

6. Procédé selon la revendication 1 ou 3, **caractérisé** en ce que l'on utilise, comme solvants, le chloropropane, le chlorure de méthylène, le chloroforme ou l'o-chlorotoluène, tel quel ou en mélange avec d'autres solvants polaires.

7. Procédé selon l'une quelconque des revendications 1 à 6, **caractérisé** en ce que la modification de la membrane de dialyse est précédée d'un traitement avec un solvant polaire.

8. Procédé selon la revendication 7, **caractérisé** en ce que sont ôtées simultanément d'une membrane à fibres creuses, par lavage avec le solvant polaire, des quantités restantes, éventuellement présentes, de liquide formant l'espace creux.

9. Dispositif pour la réalisation du procédé, constitué d'un système de canalisation (14), qui est relié à un ou plusieurs réservoirs pour des liquides (1, 2, 3, 4) et/ou des gaz (6) et à un ou plusieurs modules à membrane (13), et d'un dispositif de circulation (16) pour le maintien d'une circulation de liquide, **caractérisé** en ce que le système de canalisation (14) est une canalisation en boucle, à laquelle sont raccordés l'un après l'autre, par des dispositifs de dosage (17), les réservoirs (1, 2, 3, 4, 6), et en ce que les dispositifs de dosage (17) sont conçus de telle manière que les réactifs, les solvants et/ou les produits de lavage peuvent être conduits par la canalisation en boucle, à des moments prédéterminés et en une quantité prédéterminée, à au moins un compartiment des modules à membrane (13).

10. Dispositif selon la revendication 9, **caractérisé** en ce que plusieurs modules à membrane sont introduits dans un logement (12) muni d'ouvertures pour l'écoulement, et un ou plusieurs logements (12) sont insérés dans un récipient de réaction (9), qui est intercalé dans la canalisation en boucle (14).

11. Dispositif selon la revendication 10, **caractérisé** en ce que les modules à membrane sont disposés dans le logement (12) de façon à être tassés le plus possible.

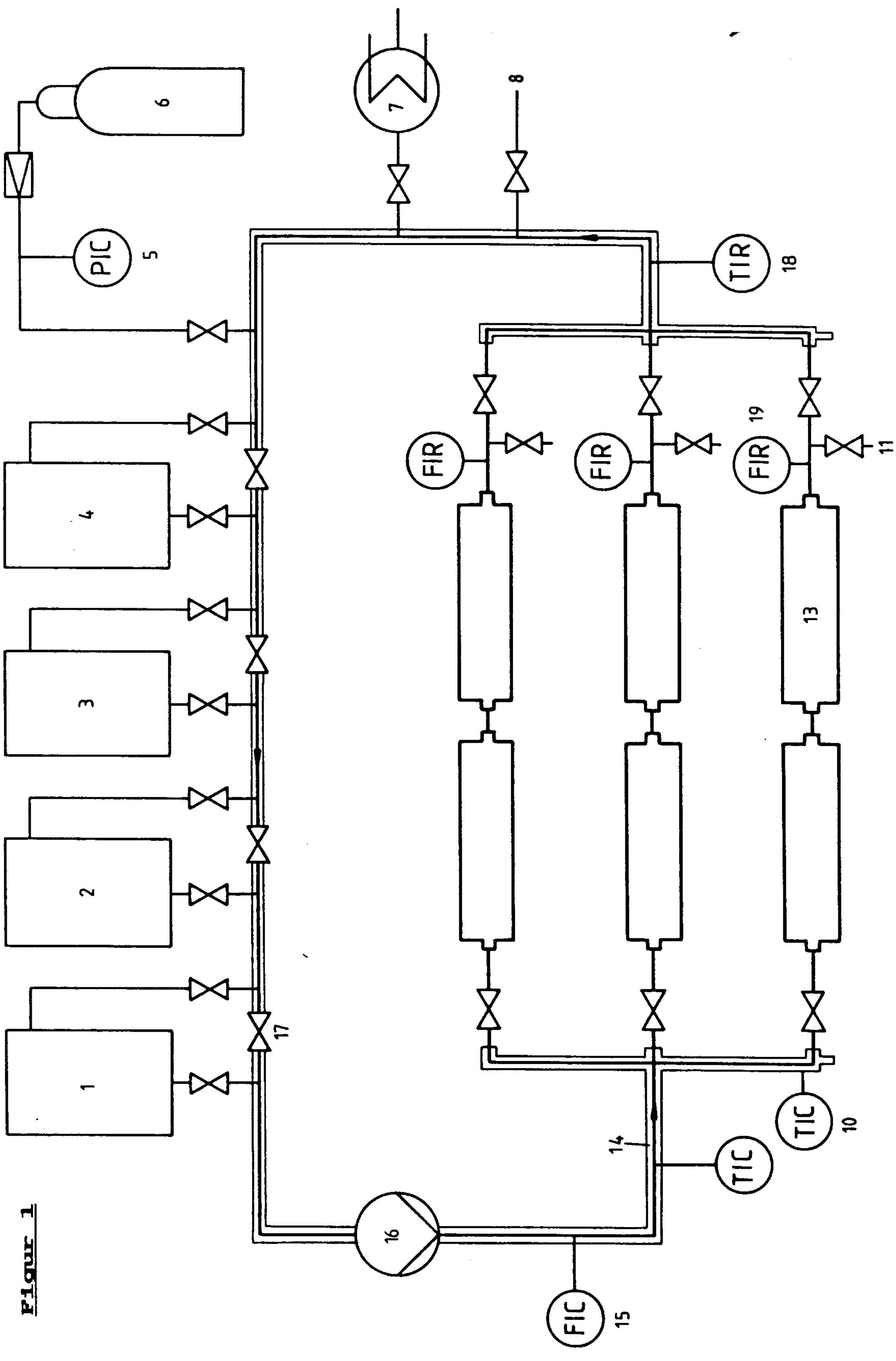
PIC
5
6
7
8
1
2
3
4
17
TIR
18
FIR
FIR
FIR
19
11
13
TIC
10
TIC
14
16
FIC
15

Figur 1

Figur 2

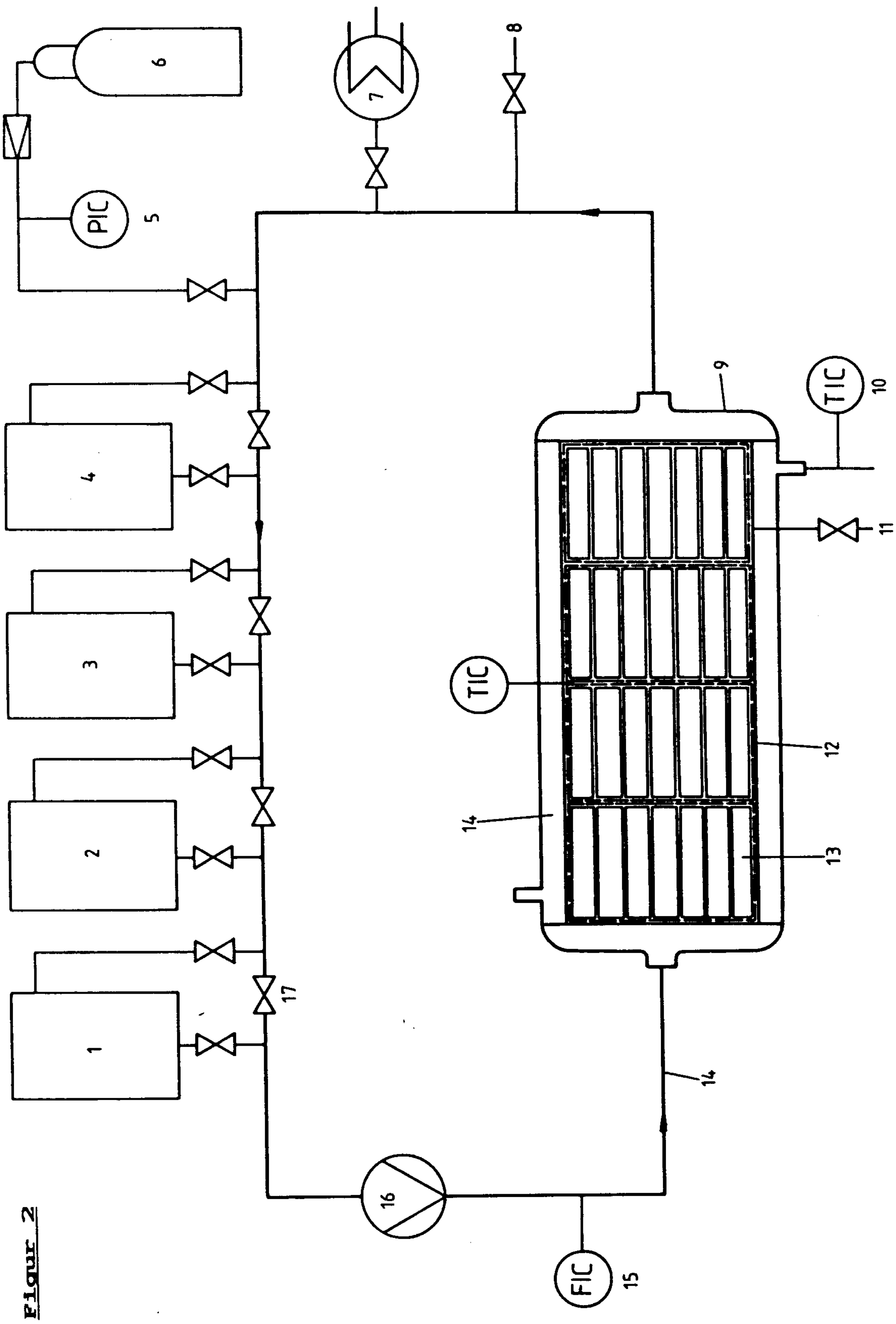

Figur 3

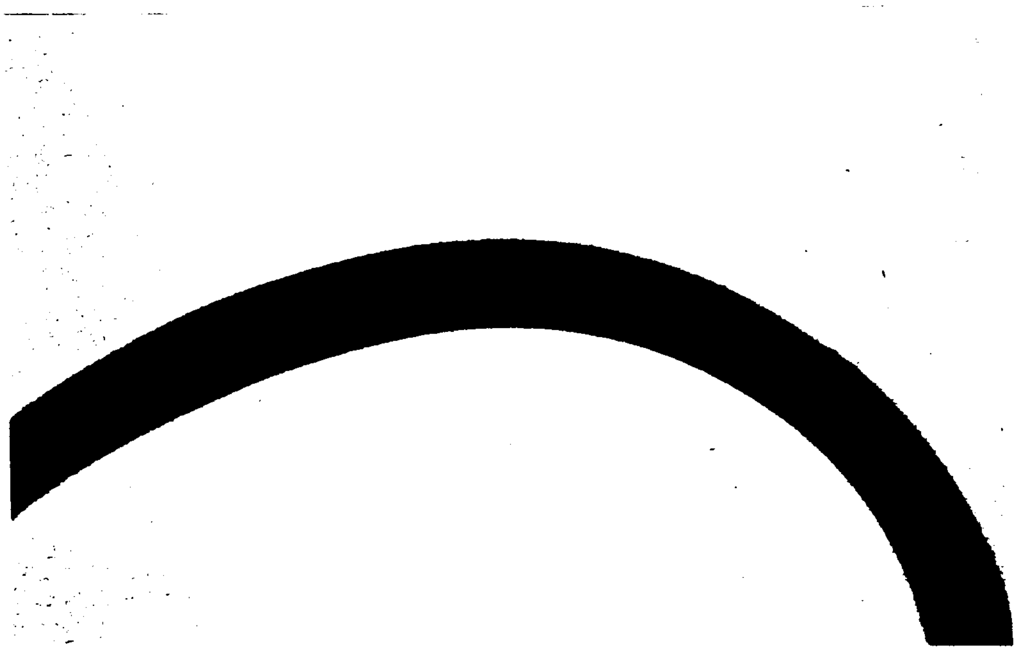

Figur 4

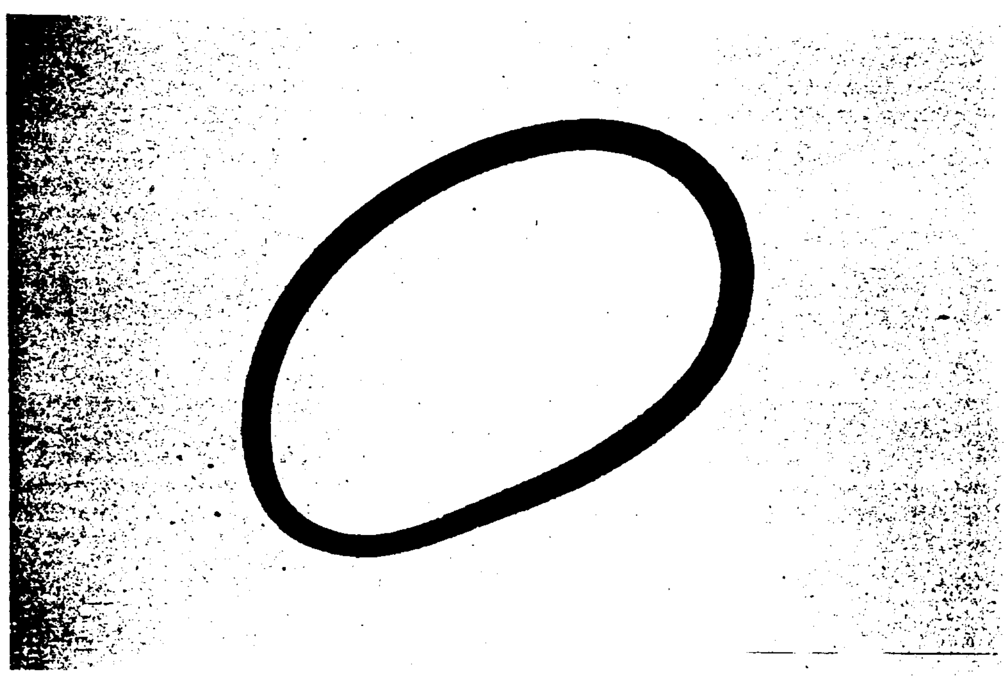

**Figur 5**

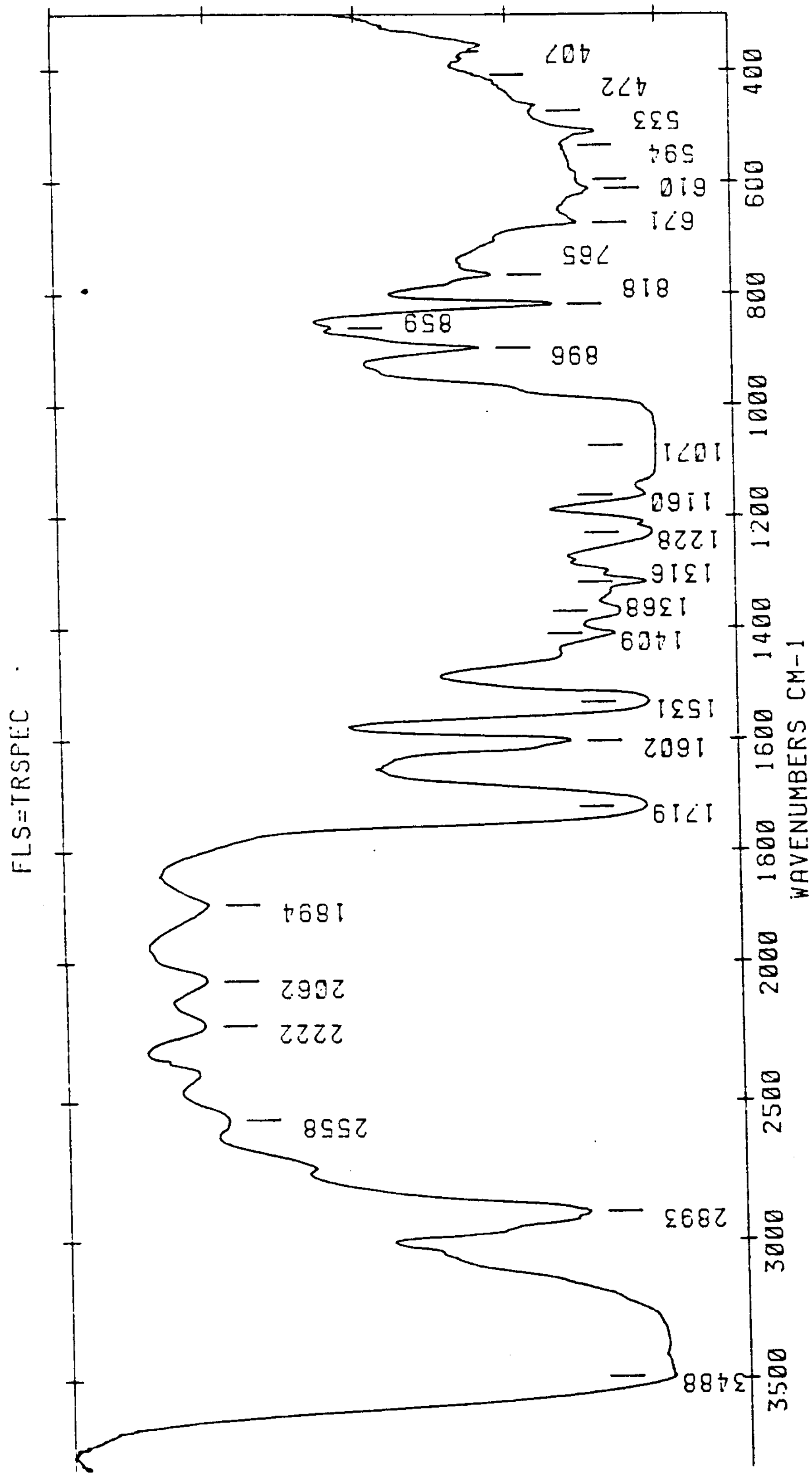
FLS=TRSPEC
WAVENUMBERS CM-1
400
600
800
1000
1200
1400
1600
1800
2000
2500
3000
3500
407
472
533
594
610
671
765
818
859
896
1071
1160
1228
1316
1368
1409
1531
1602
1719
1894
2062
2222
2558
2893
3488